# EUROPÄISCHE PATENTSCHRIFT

(11) **EP 3 454 754 B1**
(45) Veröffentlichungstag und Bekanntmachung des Hinweises auf die Patenterteilung: **28.07.2021**
(21) Anmeldenummer: 17729070.7
(22) Anmeldetag: 02.06.2017
(51) Int. Cl.: A61B 6/10, A61B 6/00, G05D 1/02

(54) **VERFAHREN ZUM BETRIEB EINER WENIGSTENS TEILWEISE AUTONOM BEWEGTEN, MOBILEN MEDIZINTECHNISCHEN EINHEIT UND MOBILE MEDIZINTECHNISCHE EINHEIT**
METHOD FOR OPERATING AN AT LEAST PARTIALLY AUTONOMOUSLY MOVING, MOBILE MEDICAL UNIT, AND MOBILE MEDICAL UNIT
PROCÉDÉ POUR FAIRE FONCTIONNER UNE UNITÉ MÉDICO-TECHNIQUE MOBILE, DÉPLACÉE DE MANIÈRE AU MOINS PARTIELLEMENT AUTONOME, ET UNITÉ MÉDICO-TECHNIQUE MOBILE

(30) Priorität: 04.07.2016 DE 102016212077
(43) Veröffentlichungstag der Anmeldung: 20.03.2019
(73) Patentinhaber: Siemens Healthcare GmbH, 91052 Erlangen (DE)
(72) Erfinder: DIRAUF, Franz, 96231 Bad Staffelstein (DE); SCHMIDT, Verena, 92681 Erbendorf (DE)
(86) Internationale Anmeldenummer: PCT/EP2017/063419
(87) Internationale Veröffentlichungsnummer: WO 2018/007076

(56) Entgegenhaltungen:
- WO-A2-2011/146259
- DE-A1-102008 046 346
- DE-A1-102015 212 886
- DE-T5-112011 105 449

## Beschreibung

Die Erfindung betrifft ein Verfahren zum Betrieb einer wenigstens teilweise autonom bewegten, mobilen medizintechnischen Einheit, aufweisend wenigstens eine Erfassungseinrichtung zur Detektion von möglichen Kollisionsobjekten im Umfeld der Einheit und eine die Erfassungsdaten der Erfassungseinrichtung auswertende Steuereinrichtung, die zum wenigstens teilweise autonomen Betrieb zur Bewegung der Einheit unter Realisierung einer Kollisionsschutzfunktion ausgebildet ist. Daneben betrifft die Erfindung eine mobile medizintechnische Einheit.

Im medizintechnischen Bereich wurden bereits Einheiten vorgeschlagen, die sich, beispielsweise in einem Krankenhaus und/oder in einer Radiologieabteilung, selbsttätig, das heißt autonom, bewegen sollen, beispielsweise zu einem Zielort. Dabei ist es im medizintechnischen Bereich wesentlich, Menschen, insbesondere Patienten, deren Besucher und das medizinische Personal, sowie andere, gegebenenfalls wertvolle medizintechnische Einrichtungen und Geräte zu schützen. Daher sind derartige autonom fahrende medizintechnische Einheiten üblicherweise mit einer Kollisionsschutzfunktion versehen, wobei üblicherweise eine Sensorik der medizintechnischen Einheit bzw. eine externe Sensorik genutzt wird, um Objekte im Bewegungsweg der mobilen medizintechnischen Einheit zu detektieren und durch eine entsprechende Anpassung des autonomen Betriebs der mobilen medizintechnischen Einheit eine Kollision zu vermeiden. Insbesondere sind Kollisionsschutzfunktionen, die durch Steuereinrichtungen von mobilen medizintechnischen Einheiten realisiert werden, meist so ausgebildet, dass bei Eintritt eines möglichen Kollisionsobjekts in einen gegebenenfalls gestaffelten Warnbereich langsamer gefahren wird bzw. gestoppt wird. Aus der DE 10 2015 212 886 A1 ist beispielsweise bekannt, dass ein C-Bogen Röntgengerät (teil-)autonom betrieben werden kann, wobei Sensoren aus Gründen der Sicherheit eine Kollision erkennen und/oder vermeiden.

Problematisch ist hierbei allerdings, dass in manchen Situationen, in denen die mobile medizintechnische Einheit mit anderen Objekten interagieren soll, eine Kollision mit diesem Objekt erwünscht sein kann. Fährt beispielsweise ein autonom bewegbarer C-Bogen als mobile medizintechnische Einheit an eine Patientenlagerungseinrichtung heran, wird sich die Patientenlagerungseinrichtung im Kollisionsbereich bzw. gestaffelten Warnbereich befinden. Ein anderes Beispiel ist der Andockvorgang einer Patientenlagerungseinrichtung als mobile medizintechnische Einheit an eine Magnetresonanzeinrichtung oder sonstige Bildgebungseinrichtung. Hier muss beispielsweise der autonome Betrieb zur Bewegung beendet werden und die Interaktion manuell initiiert werden. Ein weiterer Nachteil bekannter Kollisionsschutzfunktionen ist, dass die Reaktionen der mobilen medizintechnischen Einheit auf ein mögliches Kollisionsobjekt im zukünftigen Bewegungsweg auf das die maximale Sicherheit erfordernde mögliche Kollisionsobjekt ausgelegt werden muss, was unter Umständen dazu führen kann, dass die autonome Bewegung der mobilen medizintechnischen Einheit gänzlich abgebrochen wird.

Der Erfindung liegt daher die Aufgabe zugrunde, ein demgegenüber verbessertes Verfahren zum Betrieb einer mobilen medizintechnischen Einheit anzugeben, welches insbesondere eine Interaktion mit anderen Objekten erlaubt und/oder einen größeren Nutzungsbereich für die autonome Bewegung eröffnet.

Zur Lösung dieser Aufgabe ist bei einem Verfahren der eingangs genannten Art erfindungsgemäß vorgesehen, dass ein durch die Erfassungsdaten beschriebenes mögliches Kollisionsobjekt durch Auswertung der Erfassungsdaten klassifiziert wird und die Klassifizierung bei dem autonomen Betrieb der Einheit, insbesondere bei der Berechnung einer abzufahrenden Trajektorie, berücksichtigt wird.

Dabei können mögliche Kollisionsobjekte alle Objekte im (erfassten) Umfeld der mobilen medizintechnischen Einheit sein, wobei der Begriff des möglichen Kollisionsobjekts aber auch auf solche beschränkt werden kann, die tatsächlich mit einer aktuell geplanten und/oder prädizierten, abzufahrenden Trajektorie konfliktieren. Beispielsweise kann zur Selektion als relevantes Umgebungsobjekt, also mögliches Kollisionsobjekt, das klassifiziert werden soll, ein eine Kollisionswahrscheinlichkeit und/oder eine stattfindende prädizierte Kollision beschreibender Kollisionswert mit einem Schwellwert verglichen oder anderweitig berücksichtigt werden.

Die Erfindung schlägt mithin vor, eine mobile medizintechnische Einheit mit der Möglichkeit zu versehen, ein potentielles Kollisionsobjekt zu identifizieren und/oder zu klassifizieren. Auf diese Weise entsteht die Möglichkeit, spezielle Verhaltensregeln bezüglich bestimmter Objektklassen umzusetzen und somit einen flexibleren, deutlich verbesserten autonomen Betrieb zur Bewegung der mobilen medizintechnischen Einheit zu ermöglichen, nachdem auf spezielle Eigenschaften von Objekten einer Objektklasse eingegangen werden kann und/oder eine Interaktion mit Objekten einer bestimmten Objektklasse gezielt zugelassen werden kann. Dabei ist es in einfachen Anwendungsfällen bereits ausreichend, wenn die Klassifizierung eines möglichen Kollisionsobjekts als zu einer bestimmten Objektklasse gehörig oder nicht zu dieser Objektklasse gehörig (also zu einer aller anderen Objekte umfassenden Objektklasse gehörig) identifiziert wird. Beispielsweise kann im Fall einer anzudockenden Patientenlagerungseinrichtung, insbesondere eines Patiententisches, als mobile medizintechnische Einheit die bestimmte Objektklasse Objekte umfassen, an die die Patientenlagerungseinrichtung andocken soll, beispielsweise Magnetresonanzeinrichtungen. Für diese kann dann beispielsweise die Interaktion zugelassen werden, indem im einfachen Fall die Kollisionsschutzfunktion für dieses mögliche Kollisionsobjekt außer Kraft gesetzt wird. So würden nicht der bestimmten Objektklasse angehörende Objekte geschützt werden, beispielsweise durch Umfahren, während die Interaktion nicht gestört wird. Im allgemeinen Fall werden jedoch viele verschiedene Objektklassen vorliegen, für die spezielle Verhaltensregeln definiert werden können.

Auf diese Weise ergibt sich mit besonderem Vorteil eine kontextsensitive Bahnplanung und/oder kontextsensitive autonome Bewegung, das bedeutet, die Reaktion der mobilen medizintechnischen Einheit auf mögliche Hindernisse im Verfahrbereich ist abhängig von der Klassifizierung des möglichen Kollisionsobjektes und insbesondere seinen Eigenschaften. Der Anwendungsbereich für den autonomen Betrieb mobiler medizintechnischer Einheiten wird erweitert, indem Interaktionen erlaubt werden können und/oder nicht zwangsläufig von der Objektklasse ausgegangen werden muss, für die das größte Schutzbedürfnis besteht.

Dabei sei an dieser Stelle bereits angemerkt, dass Reaktionen der mobilen medizintechnischen Einheit auf mögliche Kollisionsobjekte, gegebenenfalls auch abhängig von der Objektklasse, eine Anpassung der Geschwindigkeit, eine Berechnung einer neuen, abzufahrenden Trajektorie, und/oder einen Wartevorgang umfassen können. Auch eine Interaktion ist mittels des vorliegenden Verfahrens nun denkbar.

Vorzugsweise kann die Erfassungseinrichtung wenigstens einen bildgebenden Sensor, insbesondere eine Kamera, umfassen, wobei die Steuereinrichtung die Erfassungsdaten des bildgebenden Sensors durch eine Bildverarbeitung auswertet, und/oder eine eine Ausleseeinrichtung für einen Funkmarker, insbesondere einen RFID-Chip, des möglichen Kollisionsobjekts umfassende Erfassungseinrichtung verwendet werden. Beispielsweise kann also eine Kamera verwendet werden, deren Bilddaten als Erfassungsdaten beispielsweise durch entsprechende, grundsätzlich bekannte Bildverarbeitungsalgorithmen ausgewertet werden können, um die Klassifizierung zu ermöglichen. Besonders vorteilhaft kann es auch sein, Marker zu verwenden, die die Identifikation eines möglichen Kollisionsobjekts erlauben, beispielsweise RFID-Marker. Selbstverständlich können auch andere Erfassungseinrichtungen, insbesondere Sensoren, eingesetzt werden, um weitere Informationen über ein mögliches Kollisionsobjekt in Form von Erfassungsdaten zu sammeln, wie dies grundsätzlich bekannt ist. Bevorzugt wird es jedoch, ohnehin bereits häufig an mobilen medizintechnischen Einheiten vorgesehene Sensoren und deren Erfassungsdaten zu nutzen.

Dabei sei an dieser Stelle nochmals angemerkt, dass eine Objektklasse durchaus auch als nur ein einziges Objekt enthaltend definiert sein kann, beispielsweise, wenn die mobile medizintechnische Einheit einem bestimmten anderen medizinischen Gerät, beispielsweise einer Bildgebungseinrichtung, zugeordnet ist und nur mit dieser wechselwirken soll oder in der Lage sein soll, zwischen mehreren zugeordneten medizintechnischen Geräten zu unterscheiden. In diesem Fall ist die Klassifizierung für zumindest die Objekte solcher Objektklassen konkret als Identifikation aufzufassen.

Eine zweckmäßige Weiterbildung der Erfindung sieht vor, dass die Steuereinrichtung wenigstens eine ein Merkmal des möglichen Kollisionsobjekts beschreibende Merkmalsinformation aus den Erfassungsdaten ermittelt und das mögliche Kollisionsobjekt durch Vergleich mit das Merkmal für Objekte wenigstens einer Objektklasse beschreibenden Referenzinformationen klassifiziert. Lassen sich mithin aus den Erfassungsdaten Merkmale des möglichen Kollisionsobjekts ableiten oder sind sie gar ohnehin bereits grundlegend in diesen enthalten, beispielsweise bei Auslesen von Funkmarkern, kann die Klassifizierung durch Vergleich entsprechender Merkmalsinformationen mit Referenzinformationen erfolgen, die der Steuereinrichtung vorliegen oder durch diese zumindest abrufbar sind. Beispiele für Merkmale sind Marker an dem möglichen Kollisionsobjekt, bei denen es sich auch um optische, gezielt zur Identifikation bzw. Klassifizierung des möglichen Kollisionsobjekts mittels einer Kamera angebrachte Marker handeln kann, und/oder die geometrische Form des möglichen Kollisionsobjekts betreffende Merkmale. Selbstverständlich sind auch verschiedenste andere Merkmale denkbar, die sich insbesondere auch aus Bilddaten als Erfassungsdaten einer Kamera ableiten lassen, beispielsweise deutlich erkennbare Komponenten und dergleichen. Werden Funkmarker, insbesondere RFID-Marker, ausgelesen, können die Merkmalsinformationen auch unmittelbar in den Erfassungsdaten vorliegen, beispielsweise als eine Identifikationsinformation, eine Geräteklasseninformation und dergleichen.

Zweckmäßigerweise kann die Steuereinrichtung die Referenzinformation aus einer in einer Speichereinrichtung der mobilen medizintechnischen Einheit und/oder einer externen Recheneinrichtung gespeicherten Referenzinformationsdatenbank abrufen. Eine derartige Referenzinformationsdatenbank enthält die Referenzinformationen für alle Objektklassen, die betrachtet werden sollten, gegebenenfalls mit der Ausnahme einer Objektklasse, die anzeigt, dass das mögliche Kollisionsobjekt zu keiner der anderen Objektklassen gehört. Bevorzugt ist die Referenzinformationsdatenbank auf einer externen Recheneinrichtung, insbesondere einem Server, gespeichert, so dass eine Mehrzahl von mobilen medizintechnischen Einheiten die entsprechenden Referenzinformationen abrufen können, wobei ferner die Möglichkeit besteht, diese zentral aktuell zu halten. Selbstverständlich ist es auch denkbar, die Referenzinformationsdatenbank regelmäßig von der externen Recheneinrichtung abzurufen und lokal in der mobilen medizintechnischen Einheit vorzuhalten.

Die Steuereinrichtung passt wenigstens einen auf das Verhalten gegenüber dem möglichen Kollisionsobjekt im Rahmen des autonomen Bewegungsbetriebs bezogenen, insbesondere bei der Berechnung und/oder der Anpassung der abzufahrenden Trajektorie zu verwendenden, Betriebsparameter für mögliche Kollisionspartner wenigstens einer ausgewählten Objektklasse an.

Wie bereits angedeutet, erlaubt es das erfindungsgemäße Verfahren insbesondere, auf der Klassifizierung aufsetzend spezielle Verhaltensregeln gegenüber Objekten wenigstens einer ausgewählten Objektklasse vorzugeben und somit kontextsensitiv den automatischen Betrieb zur Bewegung flexibler und situationsangepasster zu gestalten. Beispielsweise ist es möglich, unterschiedliche Sicherheitsforderungen abzubilden, beispielsweise also von Personen einen größeren Abstand einzuhalten und langsamere Geschwindigkeiten zu wählen als für unbewegliche, statische Objekte. Auch können Interaktionen explizit zugelassen werden.

Statische bzw. robuste Objekte können eng umfahren werden bzw. zur Interaktion genutzt werden, während der Personenschutz weiterhin gewährleistet ist.

Bei einer zur Interaktion mit der mobilen medizintechnischen Einheit vorgesehene Objekte beschreibenden ausgewählten Objektklasse wird die Kollisionsschutzfunktion und/oder die Berechnung der abzufahrenden Trajektorie die vorgesehene Interaktion erlaubend angepasst. Das bedeutet, die Interaktion selber kann in den autonomen Betrieb inkludiert werden, so dass hier keine manuelle Interaktion erforderlich ist. Dies gelingt, ohne dass es erforderlich wird, Sicherheitsfunktionen zum Personenschutz und Beschädigungsschutz einzuschränken, nachdem Objekte, mit denen interagiert werden soll, durch die Klassifizierung erkannt werden können. Beispielsweise kann dann, wenn ein mögliches Kollisionsobjekt als Objekt einer Objektklasse von Objekten, mit denen interagiert werden soll, klassifiziert wird, ein Kollisionskurs aufrechterhalten werden, gegebenenfalls unter Anpassung der Geschwindigkeit, so dass beispielsweise ein Andocken einer Patientenlagerungseinrichtung an eine Magnetresonanzeinrichtung bzw. das Heranfahren eines mobilen C-Bogens an eine Patientenlagerungseinrichtung ermöglicht wird. Für zur Interaktion mit der mobilen medizintechnischen Einheit vorgesehene Objekte wird eine Berührung, insbesondere zur Durchführung der Interaktion und/oder mit einer als Betriebsparameter verwendeten zulässigen Interaktionskraft, zugelassen. Die Anpassung der Betriebsparameter bzw. allgemein des autonomen Betriebs kann also speziell auf die erfolgreiche und/oder sichere Durchführung der Interaktion durch Wahl geeigneter Annäherungsgeschwindigkeiten, geeigneter Berührungskräfte und dergleichen erfolgen.

Besonders bevorzugt ist es im Rahmen der vorliegenden Erfindung, wenn die wenigstens eine Objektklasse, für die Anpassungen der Betriebsparameter vorzunehmen sind, abhängig von einem aktuellen Betriebsmodus und/oder einem aktuellen Betriebsziel dynamisch ausgewählt wird. Das bedeutet, die ausgewählten Objektklassen müssen, zumindest nicht vollständig, mit ihren Konsequenzen vorgegeben sein, sondern es ist durchaus möglich, abhängig vom aktuellen Betriebsmodus bzw. Betriebsziel das Verhalten gegenüber möglichen Kollisionsobjekten unterschiedlicher Objektklassen zu verändern. So kann beispielsweise bei einer mobilen Patientenlagerungseinrichtung als mobile medizintechnische Einheit zunächst ein Betriebsziel sein, Röntgenaufnahmen zu tätigen, so dass an eine Röntgeneinrichtung angedockt werden soll, später jedoch sich das Betriebsziel ändern, nachdem auch eine Magnetresonanzuntersuchung durchgeführt werden soll, so dass dann das Interaktionsziel eine Magnetresonanzeinrichtung ist. Mit dem zweiten Betriebsziel wäre die Röntgeneinrichtung dann ein zu umfahrendes Hindernis, wie es die Magnetresonanzeinrichtung beim ersten Betriebsziel ist. Selbstverständlich ist es in diesem Kontext auch denkbar, dass die Anpassung in Abhängigkeit von dem aktuellen Betriebsmodus und/oder dem aktuellen Betriebsziel erfolgt. Werden beispielsweise die angepassten Betriebsparameter aus einer Parameterdatenbank abgerufen, worauf im Folgenden noch näher eingegangen werden wird, können dort auch mehrere Sätze von Betriebsparametern hinterlegt sein, die unterschiedlichen Betriebszielen und/oder Betriebsmodi zugeordnet sind. Auf diese Weise ist die Kontextsensitivität über ein verbessertes Umfeldverständnis hinaus auch auf den aktuellen Betriebszustand der mobilen medizintechnischen Einheit erweitert, so dass das Konzept der kontextsensitiven Bahnplanung bzw. Trajektorienplanung weitgehender angewendet wird.

In einer zweckmäßigen Weiterbildung kann vorgesehen sein, dass die Anpassung des Betriebsparameters in Abhängigkeit wenigstens eines ein Objekt der ausgewählten Objektklasse beschreibenden Objektparameters erfolgt. Bei dem hier genannten Objekt kann es sich dabei sowohl um ein generelles, repräsentatives Objekt der ausgewählten Objektklasse handeln als auch um ein konkretes mögliches Kollisionsobjekt, das als Mitglied der ausgewählten Objektklasse klassifiziert wird, nachdem sich Objektparameter durchaus auch aus Erfassungsdaten ableiten lassen, worauf im Folgenden noch näher eingegangen werden wird. Die Verwendung von Objektparametern im erfindungsgemäßen Verfahren erlaubt es, das Verhalten der mobilen medizintechnischen Einheit nochmals deutlich besser auf konkrete Eigenschaften des Objekts im Allgemeinen bzw. des möglichen Kollisionsobjekts im Speziellen abzustellen.

Dabei kann vorgesehen sein, dass als Objektparameter wenigstens ein eine Ausdehnung des Objekts der ausgewählten Objektklasse und/oder eine Mobilität des Objekts der ausgewählten Objektklasse und/oder ein Interaktionsbereich und/oder ein Interaktionspunkt des Objekts der ausgewählten Objektklasse und/oder eine Annäherungsrichtung an das Objekt der ausgewählten Objektklasse und/oder ein Bewegungsparameter, insbesondere eine Bewegungsrichtung, des Objekts der ausgewählten Objektklasse verwendet werden. Selbstverständlich sind auch weitere, für den autonomen Betrieb der mobilen medizintechnischen Einheit nützliche Objektparameter denkbar. Konkret kann beispielsweise vorgesehen sein, dass bei einem als bewegbar angezeigten Objekt der ausgewählten Objektklasse und einer zu einer Kollision führenden, aktuellen, abzufahrenden Trajektorie des autonomen Betriebs auf ein Wegbewegen des möglichen Kollisionsobjekts gewartet wird, insbesondere für eine auf die ausgewählte Objektklasse abgestimmte Wartezeit als Betriebsparameter. Handelt es sich bei dem möglichen Kollisionsobjekt anhand seiner Objektklasse um ein Objekt, welches sich mit hoher Wahrscheinlichkeit bewegt, kann die Entscheidung getroffen werden, dass die mobile medizintechnische Einheit wartet, bis sich das mögliche Kollisionsobjekt wegbewegt hat. Insbesondere durch zusätzliche Beobachtung anhand der Erfassungsdaten, beispielsweise durch eine Kamera, kann zudem auch die Bewegungsrichtung bestimmt werden und die kollisionsfreie Wegplanung kann sich hiernach richten. So kann beispielsweise vorgesehen sein, dass eine durch Betriebsparameter beschriebene Umfahrungstrajektorie des autonomen Betriebs für das mögliche Kollisionsobjekt in Abhängigkeit der Ausdehnung und/oder der Bewegungsrichtung als Objektparameter ermittelt wird. Auf diese Weise kann eine sichere, kollisionsfreie Umfahrung von möglichen Kollisionsobjekten erfolgen, unter Berücksichtigung einer aktuellen Bewegungsrichtung des möglichen Kollisionsobjekts sogar besonders effektiv, da es sich voraussichtlich bewegt haben wird, während sich die mobile medizintechnische Einheit ebenso weiterbewegt. Objektparameter sind ferner nützlich, wenn eine Interaktion mit dem möglichen Kollisionsobjekt vorgesehen ist. Dann kann eine zur Interaktion mit dem möglichen Kollisionsobjekt vorgesehene Interaktionstrajektorie in Abhängigkeit des Interaktionsbereichs und/oder des Interaktionspunktes und/oder der Annäherungsrichtung als Objektparameter ermittelt werden. Auf diese Weise kann beispielsweise ein Andockpunkt durch die Objektparameter genau bestimmt werden, so dass die Interaktionstrajektorie so bestimmt werden kann, dass die Annäherung aus der korrekten Richtung mit der korrekten Geschwindigkeit erfolgt und/oder eine korrekte Interaktionskraft gegeben ist. Doch auch allgemein lässt sich sagen, dass bei einer Parametrisierung von Verhaltensregeln gemäß den Eigenschaften eines möglichen Kollisionsobjekts, also den Objektparametern, Betriebsparameter wie die Annäherungsgeschwindigkeit, ein minimaler Abstand bei Annäherung oder beim Umfahren, die Wartezeit bei einer Begegnung mobilen Objekten, die maximal zulässige Interaktionskraft bei Berührung von Objekten, bei denen dies zulässig ist, und dergleichen angepasst werden können. Größe und Ausdehnung von möglichen Kollisionsobjekten geben Hinweise, um den Radius für Ausweichmanöver anzupassen, je nachdem, wie die Objektausdehnung des möglichen Kollisionsobjekts in Fahrtrichtung ist, und dergleichen. Es sei jedoch darauf hingewiesen, dass selbstverständlich zusätzlich zu den hier beschriebenen Objektparametern und der folgenden Anpassung des autonomen Betriebs im Allgemeinen bzw. von Betriebsparametern im Speziellen selbstverständlich auch objektklassenspezifische bzw. auf die Objektklasse angepasste Betriebsparameter berücksichtigt werden können, beispielsweise minimale Abstände und/oder Dynamikbegrenzungen.

Vorzugweise kann wenigstens ein Teil der Objektparameter durch Auswertung der Erfassungsdaten bezüglich des möglichen Kollisionsobjekts ermittelt werden. Auf diese Weise werden die Erfassungsdaten zweckmäßigerweise einem weiteren Verwendungszweck zugeführt, um den kontextsensitiven Betrieb zur Bewegung der mobilen medizintechnischen Einheit weiter zu verbessern. Werden Merkmalsinformationen ermittelt, lassen sich mit besonderem Vorteil dabei erhaltene Ergebnisse auch zur Ermittlung bzw. als Objektparameter weiterverwenden; zusätzliche Eigenschaften des möglichen Kollisionsobjekts können durch Bildverarbeitung oder ähnliche Datenauswertungsalgorithmen für Sensordaten unter den Erfassungsdaten bestimmt werden und/oder in aus Funkmarkern abgerufenen Erfassungsdaten vorliegen.

In einer vorteilhaften Weiterbildung der vorliegenden Erfindung ist vorgesehen, dass wenigstens ein Teil der bezüglich einer ausgewählten Objektklasse zu verwendenden Betriebsparameter und/oder wenigstens ein Teil der Objektparameter bezogen auf ein für die ausgewählte Objektklasse repräsentatives Objekt aus einer in einer bzw. der Speichereinrichtung der mobilen medizintechnischen Einheit und/oder einer bzw. der externen Recheneinrichtung gespeicherten Parameterdatenbank, die insbesondere der Referenzinformationsdatenbank entspricht, abgerufen wird. Das bedeutet, auch für die Betriebsparameter und/oder die Objektparameter kann vorgesehen sein, dass diese in einer Datenbank, hier einer Parameterdatenbank, vorgehalten werden, die lokal und/oder extern vorliegen kann. Auch in diesem Fall wird eine zumindest teilweise extern, also beispielsweise auf einem Server, vorliegende Parameterdatenbank, auf die über eine Kommunikationsverbindung zugegriffen werden kann, bevorzugt, da die darin existenten Daten dann für mehrere mobile medizintechnische Einheiten eingesetzt werden können. Besonders vorteilhaft ist es, wenn ein Vergleich mit Referenzinformationen stattfindet, wenn die Parameterdatenbank und die Referenzinformationsdatenbank als eine Gesamtdatenbank zusammengefasst sind, in der Objektklassen sowohl die Referenzinformationen als auch Betriebsparameter und/oder Objektparameter zugeordnet sind.

Als Betriebsparameter können zweckmäßigerweise, wie bereits angedeutet, ein minimal einzuhaltender Abstand und/oder eine Annäherungsgeschwindigkeit und/oder ein Annäherungsgeschwindigkeitsverlauf und/oder eine maximal zulässige Interaktionskraft verwendet werden, wobei selbstverständlich auch eine Vielzahl anderer Betriebsparameter, die insbesondere für die Planung einer abzufahrenden Trajektorie verwendet werden, denkbar sind und berücksichtigt werden können.

Allgemein wird es im Rahmen der vorliegenden Erfindung bevorzugt, dass bei einem einer Personen umfassenden Objektklasse zugeordneten möglichen Kollisionsobjekt ein gegenüber einem Normalbetriebsmodus eine verringerte Dynamik bei dem autonomen Betrieb erlaubender Sicherheitsbetriebsmodus aktiviert wird. Dabei sei darauf hingewiesen, dass die Aktivierung eines anderen Betriebsmodus selbstverständlich mit einer Anpassung von Betriebsparametern einhergeht und somit die Ausführungen bezüglich der Betriebsparameter auch hinsichtlich von Betriebsmodi analog anwendbar sind. Auf diese Weise kann ein besonderer Schutz von Personen, die sich im Umfeld der mobilen medizintechnischen Einheit befinden können, gewährleistet werden, indem Personen enthaltenden Objektklassen nicht nur größere Sicherheitsabstände zugeordnet werden können, sondern auch insgesamt ein langsamerer, vorsichtigerer Betrieb der mobilen medizintechnischen Einheit angestrebt wird. Dabei ist es besonders vorteilhaft, wenn mehrere Personen umfassende Objektklassen verwendet werden, insbesondere eine Objektklasse für Personal und/oder eine Objektklasse für Patienten und/oder eine Objektklasse für Besucher und/oder eine Objektklasse für Kinder, wobei unterschiedliche Sicherheitsbetriebsmodi für unterschiedliche Erfahrungen im Umgang mit mobilen medizintechnischen Einheiten aufweisende Personen verwendet werden. Insbesondere kann sich die Sicherheit erhöhen, je weniger Erfahrung der entsprechenden Personengruppe im Hinblick auf mobile medizintechnische Einheiten zugeordnet werden kann. Während also beispielweise Personal mobile medizintechnische Einheiten aus der täglichen Arbeit gewöhnt ist und mit diesen hervorragend umgehen kann, ist diese Gewöhnung und Erfahrung für Patienten geringer, dennoch aber in größerem Umfang gegeben. Besucher hingegen, insbesondere Kinder, können eine äußerst geringe Erfahrung im Umgang mit mobilen medizintechnischen Einheiten vorweisen, so dass durch einen diesen Personengruppen zugeordneten Sicherheitsbetriebsmodus die größtmögliche Sicherheit bereitgestellt werden kann. So kann beispielsweise hinsichtlich Kindern neben einem größeren vorgesehenen Sicherheitsabstand beim Umfahren der Kinder auch eine langsamere Geschwindigkeit vorgesehen sein, um Kinder weniger zu erschrecken und Verletzungsrisiken, insbesondere bei spielenden Kindern, zu minimieren.

Eine weitere besonders bevorzugte Weiterbildung der Erfindung sieht vor, dass mittels einer Positionsbestimmungseinrichtung die aktuelle Position der mobilen medizintechnischen Einheit innerhalb einer Betriebsumgebung ermittelt wird, wonach der aktuellen Position zugeordnete Eigenschaftsinformationen aus einer Kartendatenbank abgerufen und bei dem autonomen Betrieb der mobilen medizintechnischen Einheit berücksichtigt werden. Dabei sind Möglichkeiten zur Positionsbestimmung, wie sie auch im erfindungsgemäßen Verfahren eingesetzt werden können, grundsätzlich bekannt und dienen beispielsweise auch der grundsätzlichen Navigation in ausgedehnteren Navigationsumgebungen, beispielsweise Krankenhäusern und/oder Radiologieabteilungen. Verschiedene Möglichkeiten der Positionsbestimmung über Funk, Kameras, Marker und dergleichen lassen sich im Rahmen der vorliegenden Erfindung einsetzen. Nachdem dann aber die mobile medizintechnische Einheit innerhalb ihrer Navigationsumgebung lokalisiert werden kann, kann in einer Kartendatenbank, insbesondere lokal in der bereits erwähnten Speichereinrichtung der mobilen medizintechnischen Einheit und/oder in der externen Recheneinrichtung, eine Vielzahl weiterer Informationen bereitgestellt werden, die bei dem autonomen Betrieb der mobilen medizintechnischen Einheit berücksichtigt werden können, insbesondere bei der Planung einer abzufahrenden Trajektorie. Die Eigenschaftsinformationen können statische, für das Verhalten gegenüber anderen Objekten relevante Eigenschaften und/oder insbesondere dynamisch in der Kartendatenbank aktualisierte aktuelle Zustandsinformationen umfassen. Konkrete statische Eigenschaften können beispielsweise die Bodenhaftung und/oder den zur Verfügung stehenden Ausweichraum betreffen, während als dynamische Zustandsinformationen ein Öffnungszustand einer Tür und/oder eine aktuelle Baustelle und/oder Problemstelle beschreibende Informationen verwendet werden können. Auf diese Weise können beispielsweise kritische Stellen für Ausweichmanöver aufgrund eines besonders engen Flurs und dergleichen identifiziert werden und es können mit besonderem Vorteil auch dynamische Eigenschaften der aktuellen Umgebung betrachtet werden, beispielsweise sich gerade schließende Türen, kurzzeitige Veränderungen des zur Verfügung stehenden Navigationsraums durch bauliche und/oder handwerkliche Maßnahmen (= Baustelle), Veränderungen der Navigationsbedingungen, beispielsweise Veränderungen der Bodenhaftung durch aktuelle Bodenzustandsveränderungen, beispielsweise Nässe, und dergleichen. Auf diese Weise wird die Kontextsensitivität des autonomen Betriebs der mobilen medizintechnischen Einheit auch auf nicht an spezielle mögliche Kollisionsobjekte gebundene, allgemeine Umgebungseigenschaften erweitert, insbesondere auch aktuelle Geschehnisse, wofür mit besonderem Vorteil die Kartendatenbank auf einer externen Recheneinrichtung abgelegt ist und dort ständig aktualisiert wird.

Im Allgemeinen können, wie aus den diskutierten Beispielen auch hervorgeht, eine Vielzahl unterschiedlicher Objektklassen im Rahmen der vorliegenden Erfindung eingesetzt werden, so dass als Objektklassen wenigstens eine Personenklasse und/oder wenigstens eine Diagnostikeinrichtungsklasse und/oder wenigstens eine Patiententransporteinrichtungsklasse und/oder wenigstens eine Therapieeinrichtungsklasse verwendet werden können.

Neben dem Verfahren betrifft die Erfindung auch eine mobile medizintechnische Einheit, aufweisend wenigstens eine Erfassungseinrichtung zur Detektion von möglichen Kollisionsobjekten im Umfeld der Einheit und eine die Erfassungsdaten der Erfassungseinrichtung auswertende Steuereinrichtung, die zum wenigstens teilweise autonomen Betrieb zur Bewegung der Einheit unter Realisierung der Kollisionsschutzfunktion ausgebildet ist, welche sich dadurch auszeichnet, dass die Steuereinrichtung zur Durchführung des erfindungsgemäßen Verfahrens ausgebildet ist. Sämtliche Ausführungen bezüglich des erfindungsgemäßen Verfahrens lassen sich analog auf die erfindungsgemäße mobile medizintechnische Einheit übertragen, so dass auch mit dieser die bereits genannten Vorteile erhalten werden können. Insbesondere kann die Steuereinrichtung also eine Trajektorienermittlungseinheit für eine abzufahrende Trajektorie und eine Klassifizierungseinheit aufweisen, wobei die Klassifizierungseinheit die Erfassungsdaten auswertet und in Konsequenz Betriebsparameter zur Ermittlung der abzufahrenden Trajektorie direkt oder indirekt anpasst. Die mobile medizintechnische Einheit kann ferner eine Speichereinrichtung und/oder eine Kommunikationseinrichtung zur Kommunikation mit einer externen Recheneinrichtung aufweisen, wobei auf der Speichereinrichtung und/oder der externen Recheneinrichtung eine Referenzinformationsdatenbank und/oder eine Parameterdatenbank und/oder eine Kartendatenbank, bevorzugt eine alle diese Datenbanken zusammenfassende Gesamtdatenbank, abgespeichert sein können.

Es sei insgesamt zur vorliegenden Erfindung noch angemerkt, dass die mobile medizintechnische Einheit selbstverständlich zur autonomen Bewegung insbesondere innerhalb von Gebäuden vorgesehen ist, beispielsweise in Arztpraxen und/oder Radiologieabteilungen und/oder Krankenhäusern als Navigationsumgebung. Bei der mobilen medizintechnischen Einheit kann es sich bevorzugt um eine Patientenlagerungseinrichtung und/oder eine Patiententransporteinrichtung und/oder eine Komponente einer medizinischen Bildgebungseinrichtung und/oder einen mobilen medizintechnischen Roboter handeln.

Weitere Vorteile und Einzelheiten der vorliegenden Erfindung ergeben sich aus den im Folgenden beschriebenen Ausführungsbeispielen sowie anhand der Zeichnung. Dabei zeigen:
Fig. 1 eine erfindungsgemäße mobile medizintechnische Einheit,
Fig. 2 einen Ablaufplan eines Ausführungsbeispiels des erfindungsgemäßen Verfahrens,
Fig. 3 eine erste Navigationssituation und
Fig. 4 eine zweite Navigationssituation.

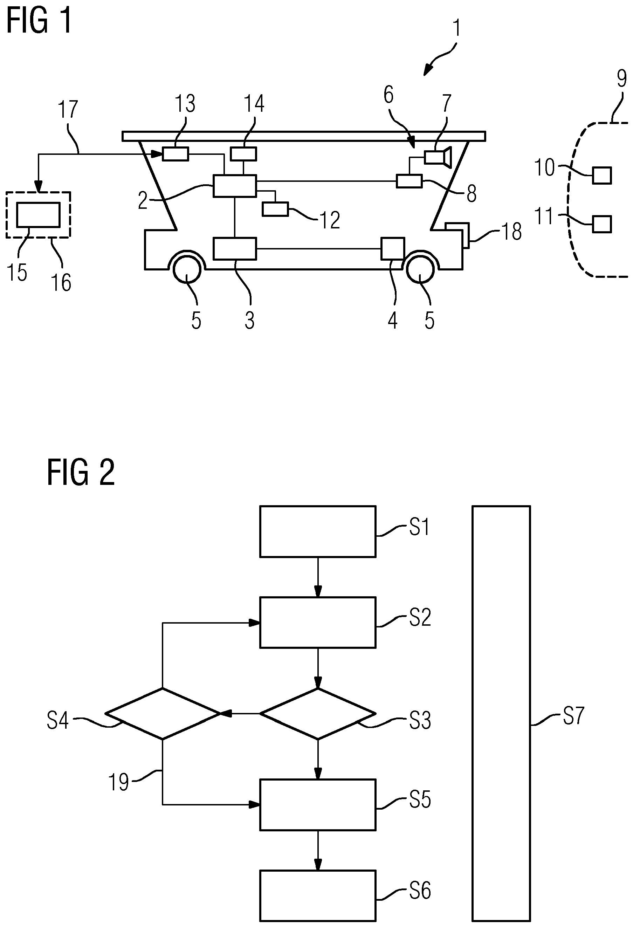

Fig. 1 zeigt eine Prinzipskizze eines Ausführungsbeispiels einer mobilen medizintechnischen Einheit 1, bei der es sich insbesondere um eine mobile Patientenlagerungseinrichtung und/oder Patiententransporteinrichtung handelt, beispielsweise einen mobilen Patiententisch, der zum wenigstens teilweise autonomen Bewegungsbetrieb ausgebildet ist.

Hierzu weist die mobile medizintechnische Einheit 1 eine Steuereinrichtung 2 auf, die zur Ermittlung einer abzufahrenden Trajektorie und zur entsprechenden Ansteuerung von Antriebsmitteln 3 und Lenkmitteln 4 für Räder 5 der Einheit 1 ausgebildet ist. Zur Erfassung des Umfelds, in dem die mobile medizintechnische Einheit 1 bewegt wird, weist sie ferner Erfassungseinrichtungen 6 auf, wobei vorliegend eine Kamera 7 als bildgebender Sensor und eine RFID-Ausleseeinrichtung 8 für einen an einem hier nur angedeuteten potentiellen Kollisionsobjekt 9 befindlichen Funkmarker 10, hier einen RFID-Chip, bezeigt sind. Mögliche Kollisionsobjekte 9 können ferner auch optische Marker 11 aufweisen, die durch Bildverarbeitung der mit der Kamera 7 aufgenommenen Bilddaten als Erfassungsdaten erkennbar sind. Zur Navigation in der Navigationsumgebung, vorliegend dem Inneren eines Gebäudes, beispielsweise eines Krankenhauses oder einer Arztpraxis, nutzt die Steuereinrichtung 2 ferner Daten einer Positionsbestimmungseinrichtung 12.

Die vorliegend ja als Patientenlagerungseinrichtung und/oder Patiententransporteinrichtung ausgebildete mobile medizintechnische Einheit 1 kann an verschiedene medizinische Bildgebungseinrichtungen, vorliegend beispielhaft eine Magnetresonanzeinrichtung, andocken, wofür sie entsprechend ferner Andockmittel 18 umfasst, die mit einer entsprechenden Andockeinrichtung wechselwirken.

Um einen kontextsensitiven autonomen Bewegungsbetrieb zu ermöglichen, insbesondere also bei der Bahnplanung, also der Ermittlung der abzufahrenden Trajektorie, nicht nur im Rahmen einer Kollisionsschutzfunktion allgemein andere Objekte zu beachten, sondern deren spezielle Eigenschaften und die Eigenschaften der Umgebung, wie sie aktuell vorliegt, berücksichtigen zu können, hat die Steuereinrichtung 2 ferner über eine Kommunikationseinrichtung 13 oder in einer Speichereinrichtung 14 Zugriff auf eine Gesamtdatenbank 15, die vorliegend als auf einer externen Recheneinrichtung 16 gespeichert gezeigt ist, zu der mittels der Kommunikationseinrichtung 13 eine drahtlose Kommunikationsverbindung 17 aufgebaut werden kann.

Die Gesamtdatenbank umfasst neben einer Kartendatenbank, die Eigenschaftsinformationen zu statischen und dynamischen Eigenschaften des aktuell befahrenen Umfelds enthält, auch eine kombinierte Parameter- und Referenzinformationsdatenbank, in der einer bestimmten Anzahl von Objektklassen Referenzinformationen zugeordnet sind, die die Steuereinrichtung 2 zur Zuordnung eines detektierten möglichen Kollisionsobjekts 9 zu einer Objektklasse mit aus den Erfassungsdaten der Erfassungseinrichtungen 6 abgeleiteten Merkmalsinformationen vergleichen kann. Ferner enthält die kombinierte Parameter- und Referenzinformationsdatenbank wenigstens einen Satz von Werten für Objekte der Objektklasse zu verwendender Betriebsparameter sowie Eigenschaften eines repräsentativen Objekts der Objektklasse beschreibende Objektparameter, aus denen sich gegebenenfalls Betriebsparameter ableiten lassen.

Die Steuereinrichtung 2 ist zur Durchführung eines Ausführungsbeispiels des erfindungsgemäßen Verfahrens ausgebildet, wie es mithilfe des Ablaufplans der Fig. 2 näher erläutert werden soll. Fig. 2 zeigt das Vorgehen, wenn durch die Erfassungseinrichtungen 6 ein mögliches Kollisionsobjekt 9 im zukünftigen Bewegungsweg der mobilen medizintechnischen Einheit 1, also entlang der abzufahrenden Trajektorie, ermittelt wird.

Hierzu werden in einem Schritt S1 zunächst die Erfassungsdaten genutzt, um daraus Merkmalsinformationen zu ermitteln, die wenigstens ein Merkmal des möglichen Kollisionsobjekts 9 beschreiben. Merkmalsinformationen können beispielsweise durch Bildverarbeitung aus Bilddaten der Kamera 7 abgeleitete geometrische Formgebungen sowie andere optisch auffällige Merkmale, beispielsweise Marker 11, beschreibende Merkmalsinformationen genauso wie durch Auslesen des Funkmarkers 10 gewonnene Merkmalsinformationen, beispielsweise Identifikationsinformationen, Geräteklasseninformationen und dergleichen, sein. Bei der Ermittlung von Merkmalsinformationen aus Bilddaten oder allgemeinen Sensordaten sowie sonstigen Erfassungsdaten können im Übrigen auch bereits später zu berücksichtigende Objektparameter, beispielsweise die Ausdehnung und/oder Größe betreffend, abgeleitet werden.

In einem Schritt S2 werden die Merkmalsinformationen mit entsprechenden, aus der Gesamtdatenbank 15 für eine zu überprüfende Objektklasse abgerufenen Referenzinformationen verglichen. Ist, vgl. Schritt S3, keine Übereinstimmung gegeben, kann in einem Schritt S4 überprüft werden, ob noch weitere Objektklassen vorliegen, bei denen ein Vergleich stattfinden soll, wobei dann wieder zu Schritt S2 verzweigt wird. Wird allerdings im Schritt S4 festgestellt, dass das mögliche Kollisionsobjekt 9 aufgrund der fehlgeschlagenen Vergleiche scheinbar keiner der in der Gesamtdatenbank 15 spezifisch erfassten Objektklassen zugeordnet ist, wird das mögliche Kollisionsobjekt gemäß dem Pfeil 19 einer Objektklasse verbleibender Objekte zugeordnet; hat sich im Vergleich jedoch schon vorher herausgestellt, vgl. Schritt S3, dass eine hinreichende Übereinstimmung mit Referenzinformationen einer bestimmten Referenzklasse vorliegt, wird das mögliche Kollisionsobjekt 9 im Schritt S5 dieser Objektklasse zugeordnet. Nachdem Objektklassen auch nur ein Objekt enthalten können, ist als Spezialform der Klassifizierung mithin auch eine Identifizierung möglich.

Nachdem das mögliche Kollisionsobjekt 9 im Schritt S5 klassifiziert wurde, erfolgt in einem Schritt S6 die Anpassung des autonomen Bewegungsbetriebs aufgrund der für das mögliche Kollisionsobjekt 9 ermittelten Objektklasse. Bei dieser Anpassung wird im Übrigen auch ein aktueller Betriebsmodus bzw. ein aktuelles Betriebsziel der mobilen medizintechnischen Einheit 1 berücksichtigt, beispielsweise, dass gerade mittels der Andockmittel 18 an eine Magnetresonanzeinrichtung angedockt werden soll, welche ein Objekt einer eigenen Objektklasse von Magnetresonanzeinrichtungen darstellt.

Zunächst werden die der Objektklasse des möglichen Kollisionsobjekts 9 zugeordneten Betriebsparameter und Objektparameter aus der Gesamtdatenbank 15 abgerufen. Liegen mehrere Sätze von Werten für Betriebsparameter vor, handelt es sich dabei üblicherweise um einen Standardsatz und einen Interaktionssatz. Zeigt der aktuelle Betriebsmodus bzw. das aktuelle Betriebsziel an, dass eine Interaktion mit Objekten dieser Objektklasse gewünscht ist, wird der Interaktionssatz von Betriebsparametern gewählt und eingestellt. Ansonsten wird der Standardsatz benutzt. Betriebsparameter können dabei beispielsweise einen minimal einzuhaltenden Abstand, eine Annäherungsgeschwindigkeit, einen Annäherungsgeschwindigkeitsverlauf, eine maximal zulässige Interaktionskraft und dergleichen umfassen. Sie werden mithin konkret bei der Ermittlung der abzufahrenden Trajektorie der mobilen medizintechnischen Einheit 1 berücksichtigt, was jedoch auch für die Eigenschaften des möglichen Kollisionsobjekts 9 der Objektklasse beschreibenden Objektparameter gilt, bei denen es sich beispielsweise um eine Ausdehnung, eine Mobilität, einen Interaktionsbereich, einen Interaktionspunkt, eine Annäherungsrichtung, oder einen Bewegungsparameter, insbesondere eine Bewegungsrichtung, handeln kann. Dabei sei nochmals darauf hingewiesen, dass die Objektparameter insbesondere wenigstens teilweise auch aus den Erfassungsdaten bestimmt werden. Die durch die Objektparameter konkret beschriebenen Eigenschaften des möglichen Kollisionsobjekts werden also ebenso bei der Ermittlung der abzufahrenden Trajektorie berücksichtigt, resultieren also ebenso in bestimmten für die Präsenz des möglichen Kollisionsobjekts 9 der Objektklasse angepassten Betriebsparametern.

Es sei angemerkt, dass das Verfahren gemäß Fig. 2 selbstverständlich immer dann durchgeführt wird, wenn ein neues mögliches Kollisionsobjekt detektiert wird. Selbstverständlich können auch mehrere klassifizierte mögliche Kollisionsobjekte 9 in den autonomen Bewegungsbetrieb Eingang finden.

Zur Auswirkung der angepassten Betriebsparameter auf den autonomen Bewegungsbetrieb der mobilen medizintechnischen Einheit 1 existieren eine Vielzahl an Möglichkeiten. Beispielsweise kann bei einem als bewegbar angezeigten Objekt der Objektklasse des möglichen Kollisionsobjekts 9 und einer zu einer Kollision führenden, aktuellen, abzufahrenden Trajektorie auf ein Wegbewegen des möglichen Kollisionsobjekts 9 gewartet werden. Ein hierbei nützlicher Betriebsparameter kann eine Wartezeit für die spezielle Objektklasse umfassen, die abbildet, wie wahrscheinlich eine Bewegung des möglichen Kollisionsobjekts 9 ist. In einem weiteren Beispiel kann die bisherige abgefahrene Trajektorie geändert werden, um eine Umfahrungstrajektorie für das mögliche Kollisionsobjekt 9 zu ermitteln und dieses mithin zu umfahren, wobei neben Betriebsparametern wie dem minimal erlaubten Abstand und erlaubten Bewegungsgeschwindigkeiten als Objektparameter die Ausdehnung und die Bewegungsrichtung berücksichtigt werden können. Ein besonderer Fall ist gegeben, wenn ein zur Interaktion vorgesehenes mögliches Kollisionsobjekt 9 anhand der Klassifizierung festgestellt wurde, denn dann kann entsprechend eine Interaktionstrajektorie in Abhängigkeit von Parametern wie Interaktionsbereich, Interaktionspunkt, Annäherungsrichtung, Annäherungsgeschwindigkeit bzw. Annäherungsgeschwindigkeitsverlauf, maximal erlaubte Einwirkkraft und dergleichen bestimmt werden, das bedeutet, die Interaktion, im hier beschriebenen Beispiel das Andocken, kann in den autonomen Betrieb inkludiert werden.

Ein wichtiger Betriebsparameter in Bezug auf Objektklassen kann auch eine maximal zulässige Bewegungsgeschwindigkeit in einem vorgegebenen Vorgabebereich um mögliche Kollisionsobjekte einer Objektklasse sein, insbesondere bei Personenklassen. Dabei werden bevorzugt mehrere Personenklassen bzw. Personen umfassende Objektklassen verwendet, vorliegend eine Objektklasse für Personal, eine Objektklasse für Patienten, eine Objektklasse für Besucher und eine Objektklasse für Kinder, wobei die maximal zulässige Bewegungsgeschwindigkeit für Kinder, die am wenigsten Erfahrung mit mobilen medizintechnischen Einheiten 1 haben, am geringsten ist und mit zunehmender Erfahrung im Umgang mit mobilen medizintechnischen Einheiten 1 ansteigen kann, so dass insgesamt die maximal erlaubte Bewegungsgeschwindigkeit für Personal größer ist als für Patienten, welche wiederum größer ist als für Besucher, welche wiederum größer ist als für Kinder.

Es sei an dieser Stelle noch angemerkt, dass während dem autonomen Bewegungsbetrieb ständig, wie durch den Schritt S7 in Fig. 2 angedeutet, auch die Kartendatenbank als Teil der Gesamtdatenbank 15 genutzt wird. Nachdem dank der Positionsbestimmungseinrichtung 12 die Position der Einheit 1 in der ihr zugeordneten Navigationsumgebung bekannt ist, können statische Eigenschaften und dynamische Eigenschaften der Umgebung an der aktuellen Position berücksichtigt werden, beispielsweise, ob sich eine vorausliegende Tür gerade schließt, ob eine Baustelle vorliegt, ein für Ausweichmanöver eher unkritischer Engpass vorliegt und dergleichen.

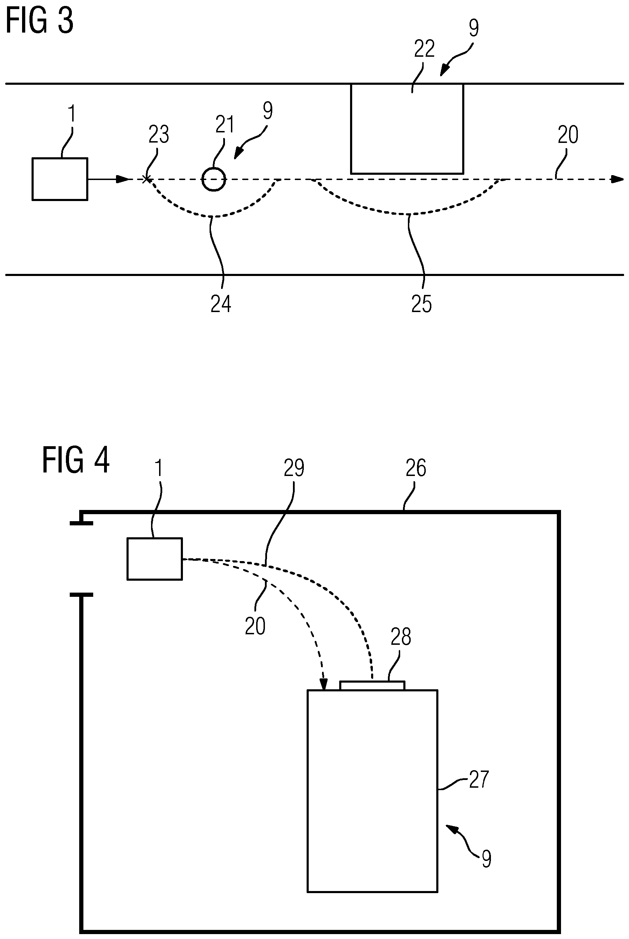

Die Figuren 3 und 4 zeigen beispielhaft Navigationssituationen, in denen sich die mobile medizintechnische Einheit 1 befinden kann.

Fig. 3 zeigt einen Fall, in dem die mobile medizintechnische Einheit 1 entlang ihrer geplanten, abzufahrenden Trajektorie 20 zwei möglichen Kollisionsobjekten 9 begegnet, nämlich einer Person 21 und einer Röntgeneinrichtung 22. Wird die Person 21 detektiert und als Person (gegebenenfalls genauer als Personal) klassifiziert, schaltet die mobile medizintechnische Einheit 1 zunächst in einen Sicherheitsbetriebsmodus um, in dem die maximal erlaubte Bewegungsgeschwindigkeit reduziert ist. Sie wird an einer Position 23 in einem vorbestimmten Abstand vor der Person 21 anhalten und für eine Wartezeit warten, ob sich die Person 21 nicht wegbewegt, nachdem diese als beweglich angezeigt wird. Hat sich die Person 21 wegbewegt, kann wieder dem ursprünglichen räumlichen Verlauf der Trajektorie 20 weitergefolgt werden. Ansonsten wird eine Umfahrungstrajektorie 24 unter Einhaltung eines minimalen Abstands zur Person 21 berechnet und verwendet.

Nachdem die mobile medizintechnische Einheit 1 an eine Magnetresonanzeinrichtung andocken soll, wird sie feststellen, dass es sich bei der Röntgeneinrichtung 22 nicht um eine solche Magnetresonanzeinrichtung handelt, indem die Röntgeneinrichtung 22 entsprechend als solche einer Röntgeneinrichtungsklasse zugehörig klassifiziert wird. Nachdem sich die Röntgeneinrichtung 22 üblicherweise auch nicht bewegt, wird hier unmittelbar eine Umfahrungstrajektorie 25 ermittelt und genutzt, die gegebenenfalls enger an der Röntgeneinrichtung 22 vorbeiführen kann, deren Ausdehnungen/Größe durch die Objektparameter auch bekannt ist.

In Fig. 4 ist in einer weiteren Navigationssituation die mobile medizintechnische Einheit 1 in einem Raum 26 angekommen, der eine Magnetresonanzeinrichtung 27 als mögliches Kollisionsobjekt 9 entlang der aktuell geplanten, abzufahrenden Trajektorie 20 enthält. Nachdem die mobile medizintechnische Einheit 1 mittels ihrer Andockmittel 18 an eine Magnetresonanzeinrichtung 27 ankoppeln soll, indem eine dortige Andockeinrichtung 28 genutzt wird, wird nach Klassifizierung der Magnetresonanzeinrichtung 27 als einer Magnetresonanzeinrichtungsklasse angehörig die Kollision nicht vermieden, nachdem ja eine Interaktion gerade gewünscht ist. Mit anderen Worten wird eine Kollisionsschutzfunktion in diesem Fall nicht genutzt. Stattdessen können die angepassten Betriebsparameter, wobei hier der Interaktionssatz verwendet wird, und die Objektparameter, die den Interaktionspunkt und die Annäherungsrichtung beschreiben, genutzt werden, um eine optimal geeignete Interaktionstrajektorie 29 zu ermitteln, wobei aus den Betriebsparametern des Interaktionssatzes folgt, dass eine gewisse Annäherungsgeschwindigkeit gegeben sein soll und eine bestimmte Interaktionskraft nicht überschritten werden soll. Auf diese Weise ist im autonomen Bewegungsbetrieb der mobilen medizintechnischen Einheit 1 auch ein automatisches Andocken möglich, insbesondere, ohne dass eine Kollisionsschutzfunktion dem entgegenstünde.

Es sei noch angemerkt, dass auch andere mobile medizintechnische Einheiten 1 denkbar sind, beispielsweise autonom bewegbare Komponenten von Bildgebungseinrichtungen, insbesondere mobile C-Bögen und dergleichen.

Obwohl die Erfindung im Detail durch das bevorzugte Ausführungsbeispiel näher illustriert und beschrieben wurde, so ist die Erfindung nicht durch die offenbarten Beispiele eingeschränkt und andere Variationen können vom Fachmann hieraus abgeleitet werden, ohne den Schutzumfang der Erfindung zu verlassen.

### Bezugszeichenliste

- 1: mobile medizintechnische Einheit
- 2: Steuereinrichtung
- 3: Antriebsmittel
- 4: Lenkmittel
- 5: Rad
- 6: Erfassungseinrichtung
- 7: Kamera
- 18: RFID-Ausleseeinrichtung
- 9: Kollisionsobjekt
- 10: Funkmarker
- 11: optischer Marker
- 12: Positionsbestimmungseinrichtung
- 153: Kommunikationseinrichtung
- 14: Speichereinrichtung
- 15: Gesamtdatenbank
- 16: Recheneinrichtung
- 17: Kommunikationsverbindung
- 208: Andockmittel
- 19: Pfeil
- 20: Trajektorie
- 21: Person
- 22: Person
- 223: Position
- 24: Umfahrungstrajektorie
- 25: Umfahrungstrajektorie
- 26: Raum
- 27: Magnetresonanzeinrichtung
- 308: Andockeinrichtung
S1 - S6 Schritt
- 35:

## Patentansprüche

1. Verfahren zum Betrieb einer wenigstens teilweise autonom bewegten, mobilen medizintechnischen Einheit (1), aufweisend wenigstens eine Erfassungseinrichtung (6) zur Detektion von möglichen Kollisionsobjekten (9) im Umfeld der Einheit (1) und eine die Erfassungsdaten der Erfassungseinrichtung (6) auswertende Steuereinrichtung (2), die zum wenigstens teilweise autonomen Betrieb zur Bewegung der Einheit (1) unter Realisierung einer Kollisionsschutzfunktion ausgebildet ist, wobei ein durch die Erfassungsdaten beschriebenes mögliches Kollisionsobjekt (9) durch Auswertung der Erfassungsdaten klassifiziert wird und die Klassifizierung bei dem autonomen Betrieb der Einheit (1) berücksichtigt wird, wobei die Steuereinrichtung (2) wenigstens einen auf das Verhalten gegenüber dem möglichen Kollisionsobjekt (9) im Rahmen des autonomen Bewegungsbetriebs bezogenen Betriebsparameter für mögliche Kollisionspartner wenigstens einer ausgewählten Objektklasse anpasst, wobei bei einer zur Interaktion mit der mobilen medizintechnischen Einheit (1) vorgesehene Objekte beschreibenden ausgewählten Objektklasse die Kollisionsschutzfunktion und/oder die Berechnung der abzufahrenden Trajektorie (20) die vorgesehene Interaktion erlaubend angepasst wird, **dadurch gekennzeichnet, dass** für zur Interaktion mit der mobilen medizintechnischen Einheit (1) vorgesehene Objekte eine Berührung, insbesondere zur Durchführung der Interaktion und/oder mit einer als Betriebsparameter verwendeten zulässigen Interaktionskraft, zugelassen wird.

2. Verfahren nach Anspruch 1, **dadurch gekennzeichnet, dass** die Erfassungseinrichtung (6) wenigstens einen bildgebenden Sensor, insbesondere eine Kamera (7), umfasst, wobei die Steuereinrichtung (2) die Erfassungsdaten des bildgebenden Sensors durch eine Bildverarbeitung auswertet, und/oder eine eine Ausleseeinrichtung (8) für einen Funkmarker (10), insbesondere einen RFID-Chip, des möglichen Kollisionsobjekts (9) umfassende Erfassungseinrichtung (6) verwendet wird.

3. Verfahren nach Anspruch 1 oder 2, **dadurch gekennzeichnet, dass** die Steuereinrichtung (2) wenigstens eine ein Merkmal des möglichen Kollisionsobjekts (9) beschreibende Merkmalsinformation aus den Erfassungsdaten ermittelt und das mögliche Kollisionsobjekt (9) durch Vergleich mit das Merkmal für Objekte wenigstens einer Objektklasse beschreibenden Referenzinformationen klassifiziert.

4. Verfahren nach Anspruch 3, **dadurch gekennzeichnet, dass** als Merkmal ein Marker (10, 11) an dem möglichen Kollisionsobjekt (9) und/oder ein eine geometrische Form des möglichen Kollisionsobjekts (9) betreffendes Merkmal verwendet wird.

5. Verfahren nach Anspruch 3 oder 4, **dadurch gekennzeichnet, dass** die Steuereinrichtung (2) die Referenzinformationen aus einer in einer Speichereinrichtung (14) der mobilen medizinischen Einheit (1) und/oder einer externen Recheneinrichtung (16) gespeicherten Referenzinformationsdatenbank abruft.

6. Verfahren nach einem der vorangehenden Ansprüche, **dadurch gekennzeichnet, dass** die wenigstens eine Objektklasse, für die Anpassungen der Betriebsparameter vorzunehmen sind, abhängig von einem aktuellen Betriebsmodus und/oder einem aktuellen Betriebsziel dynamisch ausgewählt wird und/oder die Anpassung in Abhängigkeit von dem aktuellen Betriebsmodus und/oder dem aktuellen Betriebsziel erfolgt.

7. Verfahren nach einem der vorangehenden Ansprüche, **dadurch gekennzeichnet, dass** die Anpassung des Betriebsparameters in Abhängigkeit wenigstens eines ein Objekt der ausgewählten Objektklasse beschreibenden Objektparameters erfolgt.

8. Verfahren nach Anspruch 7, **dadurch gekennzeichnet, dass** als Objektparameter wenigstens ein eine Ausdehnung des Objekts der ausgewählten Objektklasse und/oder eine Mobilität des Objekts der ausgewählten Objektklasse und/oder ein Interaktionsbereich und/oder ein Interaktionspunkt des Objekts der ausgewählten Objektklasse und/oder eine Annäherungsrichtung an das Objekt der ausgewählten Objektklasse und/oder ein Bewegungsparameter, insbesondere eine Bewegungsrichtung, des Objekts der ausgewählten Objektklasse verwendet werden.

9. Verfahren nach Anspruch 8, **dadurch gekennzeichnet, dass** bei einem als bewegbar angezeigtem Objekt der ausgewählten Objektklasse und einer zu einer Kollision führenden, aktuellen, abzufahrenden Trajektorie (20) des autonomen Betriebs auf ein Wegbewegen des möglichen Kollisionsobjekts (9) gewartet wird, insbesondere für eine auf die ausgewählte Objektklasse abgestimmte Wartezeit als Betriebsparameter, und/oder eine durch Betriebsparameter beschriebene Umfahrungstrajektorie (24, 25) des autonomen Betriebs für das mögliche Kollisionsobjekt (9) in Abhängigkeit der Ausdehnung und/oder Bewegungsrichtung als Objektparameter ermittelt wird und/oder eine zur Interaktion mit dem möglichen Kollisionsobjekt (9) vorgesehene Interaktionstrajektorie (29) in Abhängigkeit des Interaktionsbereichs und/oder des Interaktionspunktes und/oder der Annäherungsrichtung als Objektparameter ermittelt wird.

10. Verfahren nach einem der Ansprüche 7 bis 9, **dadurch gekennzeichnet, dass** wenigstens ein Teil der Objektparameter durch Auswertung der Erfassungsdaten bezüglich des möglichen Kollisionsobjekts (9) ermittelt wird.

11. Verfahren nach einem der vorangehenden Ansprüche, **dadurch gekennzeichnet, dass** wenigstens ein Teil der bezüglich einer ausgewählten Objektklasse zu verwendenden Betriebsparameter und/oder wenigstens ein Teil der Objektparameter bezogen auf ein für die ausgewählte Objektklasse repräsentatives Objekt aus einer in einer bzw. der Speichereinrichtung (14) der mobilen medizintechnischen Einheit (1) und/oder einer bzw. der externen Recheneinrichtung (16) gespeicherten Parameterdatenbank, die insbesondere der Referenzinformationsdatenbank entspricht, abgerufen wird.

12. Verfahren nach einem der vorangehenden Ansprüche, **dadurch gekennzeichnet, dass** als Betriebsparameter ein minimal einzuhaltender Abstand und/oder eine Annäherungsgeschwindigkeit und/oder ein Annäherungsgeschwindigkeitsverlauf und/oder eine maximal zulässige Interaktionskraft verwendet werden.

13. Verfahren nach einem der vorangehenden Ansprüche, **dadurch gekennzeichnet, dass** bei einem einer Personen (21) umfassenden Objektklasse zugeordneten möglichen Kollisionsobjekt (9) ein gegenüber einem Normalbetriebsmodus eine verringerte Dynamik bei dem autonomen Betrieb erlaubender Sicherheitsbetriebsmodus aktiviert wird.

14. Verfahren nach Anspruch 13, **dadurch gekennzeichnet, dass** mehrere Personen (21) umfassende Objektklassen verwendet werden, insbesondere eine Objektklasse für Personal und/oder eine Objektklasse für Patienten und/oder eine Objektklasse für Besucher und/oder eine Objektklasse für Kinder, wobei unterschiedliche Sicherheitsbetriebsmodi für unterschiedliche Erfahrungen im Umgang mit mobilen medizintechnischen Einheiten aufweisende Personen (21) verwendet werden.

15. Verfahren nach einem der vorangehenden Ansprüche, **dadurch gekennzeichnet, dass** mittels einer Positionsbestimmungseinrichtung (12) die aktuelle Position der mobilen medizintechnischen Einheit (1) innerhalb einer Betriebsumgebung ermittelt wird, wonach der aktuellen Position zugeordnete Eigenschaftsinformationen aus einer Kartendatenbank abgerufen und bei dem autonomen Betrieb der mobilen medizintechnischen Einheit (1) berücksichtigt werden.

16. Verfahren nach Anspruch 15, **dadurch gekennzeichnet, dass** die Eigenschaftsinformationen statische, für das Verhalten gegenüber anderen Objekten relevante Eigenschaften und/oder insbesondere dynamisch in der Kartendatenbank aktualisierte aktuelle Zustandsinformationen umfassen.

17. Verfahren nach Anspruch 16, **dadurch gekennzeichnet, dass** die statischen Eigenschaften die Bodenhaftung und/oder den zur Verfügung stehenden Ausweichraum betreffen und/oder als Zustandsinformationen ein Öffnungszustand einer Tür und/oder eine aktuelle Baustelle und/oder Problemstelle beschreibende Informationen verwendet werden.

18. Verfahren nach einem der vorangehenden Ansprüche, **dadurch gekennzeichnet, dass** als Objektklassen wenigstens eine Personenklasse und/oder wenigstens eine Diagnostikeinrichtungsklasse und/oder wenigstens eine Patiententransporteinrichtungsklasse und/oder wenigstens eine Therapieeinrichtungsklasse verwendet werden.

19. Mobile medizintechnische Einheit (1), aufweisend wenigstens eine Erfassungseinrichtung (6) zur Detektion von möglichen Kollisionsobjekten (9) im Umfeld der Einheit (1) und eine die Erfassungsdaten der Erfassungseinrichtung (6) auswertende Steuereinrichtung (2), die zum wenigstens teilweise autonomen Betrieb zur Bewegung der Einheit (1) unter Realisierung einer Kollisionsschutzfunktion ausgebildet ist, **dadurch gekennzeichnet, dass** die Steuereinrichtung (2) zur Durchführung eines Verfahrens nach einem der vorangehenden Ansprüche ausgebildet ist.

## Claims

1. Method for operating an at least partially autonomously moving, mobile medical unit (1), which has at least one sensing device (6) for detecting possible collision objects (9) in the environment of the unit (1) and a control device (2), which evaluates the sensing data of the sensing device (6) and which is designed to at least partially autonomously operate the movement of the unit (1) while performing a collision protection function, wherein a possible collision object (9) described by the sensing data is classified by evaluating the sensing data and the classification is taken into consideration in the autonomous operation of the unit (1), wherein the control device (2) adapts at least one operating parameter relating to the behaviour towards the possible collision object (9) in the context of autonomous movement operation for possible collision partners of at least one selected object class, wherein in a selected object class describing objects intended for interaction with the mobile medical unit (1), the collision protection function and/or the calculation of the trajectory to be travelled (20) which permits the intended interaction is adapted, **characterised in that** for objects intended for interaction with the mobile medical unit (1), a contact, in particular, for performance of the interaction and/or with a permissible force of interaction used as an operating parameter, is permitted.

2. Method according to claim 1, **characterised in that** the sensing device (6) comprises at least one imaging sensor, in particular, a camera (7), wherein the control device (2) evaluates the sensing data of the imaging sensor by means of image processing, and/or a sensing device (6) comprising a readout device (8) for a radio marker (10), in particular, an RFID chip, of the possible collision object (9) is used.

3. Method according to claim 1 or 2, **characterised in that** the control device (2) determines at least one item of feature information describing a feature of the possible collision object (9) from the sensing data and classifies the possible collision object (9) by means of comparison with the reference information describing the feature for objects of at least one object class.

4. Method according to claim 3, **characterised in that** a marker (10, 11) on the possible collision object (9) and/or a feature concerning a geometric shape of the possible collision object (9) is used as a feature.

5. Method according to claim 3 or 4, **characterised in that** the control device (2) retrieves the reference information from a reference information database stored in a storage device (14) of the mobile medical unit (1) and/or an external computing device (16).

6. Method according to one of the preceding claims, **characterised in that** the at least one object class for which adaptations of the operating parameters are to be undertaken is dynamically selected depending on a current operating mode and/or a current operational aim and/or the adaptation takes place as a function of the current operating mode and/or the current operational aim.

7. Method according to one of the preceding claims, **characterised in that** the operating parameter is adapted as a function of at least one object parameter describing an object of the selected object class.

8. Method according to claim 7, **characterised in that** at least one an extension of the object of the selected object class and/or a mobility of the object of the selected object class and/or a range of interaction and/or a point of interaction of the object of the selected object class and/or an approach direction to the object of the selected object class and/or a movement parameter, in particular, a direction of movement, of the object of the selected object class are used as object parameters.

9. Method according to claim 8, **characterised in that** in an object displayed as movable of the selected object class and a current trajectory to be travelled (20) resulting in a collision of the autonomous operation, a moving away of the possible collision object (9) is awaited, in particular, for a waiting period adapted to the selected object class as an operating parameter, and/or a circumvention trajectory (24, 25) described by operating parameters of the autonomous operation for the possible collision object (9) as a function of the dimensions and/or direction of movement as object parameters is determined and/or an interaction trajectory (29) intended for interaction with the possible collision object (9) is determined as an object parameter as a function of the range of interaction and/or the point of interaction and/or the approach direction.

10. Method according to one of claims 7 to 9, **characterised in that** at least some of the object parameters are determined by evaluating the sensing data relating to the possible collision object (9).

11. Method according to one of the preceding claims, **characterised in that** at least some of the operating parameters to be used relating to a selected object class and/or at least some of the object parameters relating to an object representative of the selected object class are retrieved from a parameter database stored in a or the storage device (14) of the mobile medical unit (1) and/or an or the external computing device (16), which in particular corresponds to the reference information database.

12. Method according to one of the preceding claims, **characterised in that** a minimum distance and/or an approach speed and/or an approach speed profile and/or a maximum permissible force of interaction are used as operating parameters.

13. Method according to one of the preceding claims, **characterised in that** in a possible collision object (9) assigned to an object class comprising a person (21), a safety operating mode permitting a reduced dynamic during autonomous operation relative to a normal operating mode is activated.

14. Method according to claim 13, **characterised in that** a plurality of object classes comprising people (21) are used, in particular, an object class for personnel and/or an object class for patients and/or an object class for visitors and/or an object class for children, wherein different safety operating modes for different experiences in dealing with people (21) with mobile medical units are used.

15. Method according to one of the preceding claims, **characterised in that** the current position of the mobile medical unit (1) within an operating environment is determined by means of a position determination device (12), according to which property information assigned to the current position is retrieved from a map database and taken into consideration during the autonomous operation of the mobile medical unit (1) .

16. Method according to claim 15, **characterised in that** the property information comprises static properties relevant to behaviour towards other objects and/or in particular, current status information dynamically updated in the map database.

17. Method according to claim 16, **characterised in that** the static properties relate to traction and/or the available bypass space and/or information describing an opening status of a door and/or a current construction site and/or problem area is used as status information.

18. Method according to one of the preceding claims, **characterised in that** at least one person class and/or at least one diagnostic device class and/or at least one patient transport device class and/or at least one therapy device class are used as object classes.

19. Mobile medical unit (1) which has at least one sensing device (6) for detecting possible collision objects (9) in the environment of the unit (1) and a control device (2) which evaluates the sensing data of the sensing device (6) and which is designed to at least partially autonomously operate the movement of the unit (1) while performing a collision protection function, **characterised in that** the control device (2) is designed to perform a method according to one of the preceding claims.

## Revendications

1. Procédé pour faire fonctionner une unité (1) mobile de la technique médicale déplacée au moins en partie de manière autonome, comportant au moins un dispositif (6) de détection pour la détection d'objets (9) de collision possibles aux alentours de l'unité (1) et un dispositif (2) de commande, qui exploite les données de détection de l'unité (6) de détection et qui est constitué pour le fonctionnement au moins en partie de manière autonome pour le déplacement de l'unité (1) en réalisant une fonction de protection vis-à-vis d'une collision, dans lequel on classe en évaluant les données de détection un objet (9) de collision possible décrit par les données de détection et on prend en compte la classification dans le fonctionnement de manière autonome de l'unité (1), le dispositif (2) de commande adaptant à au moins une classe d'objets sélectionnée au moins un paramètre de fonctionnement, rapporté au comportement vis-à-vis de l'objet (9) de collision possible dans le cadre du fonctionnement de déplacement de manière autonome, pour partenaire possible de collision, dans lequel pour une classe d'objets sélectionnée, décrivant des objets prévus pour l'interaction avec l'unité (1) mobile de la technique médicale, on adapte, en permettant l'interaction prévue, la fonction de protection vis-à-vis d'une collision et/ou le calcul de la trajectoire (20) à parcourir, **caractérisé en ce que**, pour des objets prévus pour l'interaction avec l'unité (1) mobile de la technique médicale, on autorise un contact, notamment pour effectuer l'interaction et/ou avec une force d'interaction admissible utilisée comme paramètre de fonctionnement.

2. Procédé suivant la revendication 1, **caractérisé en ce que** le dispositif (6) de détection comprend au moins un capteur donnant une image, notamment un appareil (7) photographique, le dispositif (2) de commande exploitant par un traitement d'image, les données de détection du capteur donnant une image et/ou il est utilisé un dispositif (6) de détection comprenant un dispositif (8) de lecture d'un marqueur (10) radio, notamment d'une puce RFID, de l'objet (9) de collision possible.

3. Procédé suivant la revendication 1 ou 2, **caractérisé en ce que** le dispositif (2) de commande détermine à partir des données de détection au moins une information de caractéristique décrivant une caractéristique de l'objet (9) de collision possible et classe l'objet (9) de collision possible par comparaison à des informations de référence décrivant la caractéristique pour des objets d'au moins une classe d'objets.

4. Procédé suivant la revendication 3, **caractérisé en ce que** l'on utilise comme caractéristique un marqueur (10, 11) sur l'objet (9) de collision possible et/ou une caractéristique concernant la forme géométrique de l'objet (9) de collision possible.

5. Procédé suivant la revendication 3 ou 4, **caractérisé en ce que** le dispositif (2) de commande recherche les informations de référence dans une base de données d'informations de référence mise dans un dispositif (14) de mémoire de l'unité (1) mobile de la technique médicale et/ou dans un dispositif (16) informatique extérieur.

6. Procédé suivant l'une des revendications précédentes, **caractérisé en ce que** l'on choisit dynamiquement la au moins une classe d'objets, pour laquelle on peut effectuer des adaptations du paramètre de fonctionnement, en fonction d'un mode de fonctionnement en cours et/ou d'un but de fonctionnement en cours et/ou l'adaptation s'effectue en fonction du mode de fonctionnement en cours et/ou du but de fonctionnement en cours.

7. Procédé suivant l'une des revendications précédentes, **caractérisé en ce que** l'adaptation du paramètre de fonctionnement s'effectue en fonction d'au moins un paramètre d'objet décrivant un objet de la classe d'objets sélectionnée.

8. Procédé suivant la revendication 7, **caractérisé en ce que** on utilise comme paramètre d'objet au moins l'un d'une étendue de l'objet de la classe d'objets sélectionnée et/ou d'une mobilité de l'objet de la classe d'objets sélectionnée et/ou d'un domaine d'action et/ou d'un point d'interaction de l'objet de la classe d'objets sélectionnée et/ou d'une direction d'approche à l'objet de la classe d'objets sélectionnée et/ou un paramètre de déplacement, notamment une direction de déplacement, de l'objet de la classe d'objets sélectionnée.

9. Procédé suivant la revendication 8, **caractérisé en ce que**, pour un objet, indiqué comme mobile, de la classe d'objets sélectionnée et une trajectoire (20), à parcourir en cours conduisant à une collision, du fonctionnement de manière autonome, on attend un éloignement de l'objet (9) de collision possible, notamment pendant un temps d'attente adapté à la classe d'objet sélectionnée comme paramètre de fonctionnement, et/ou on détermine comme paramètre d'objet une trajectoire (24, 25) de contournement, décrite par le paramètre de fonctionnement, du fonctionnement de manière autonome de l'objet (9) de collision possible en fonction de l'étendue et/ou de la direction de déplacement et/ou on détermine comme paramètre d'objet une trajectoire (24, 25) de contournement, décrite par le paramètre de fonctionnement, du fonctionnement de manière autonome de l'objet (9) de collision possible en fonction de l'étendue et/ou de la direction de déplacement et/ou on détermine comme paramètre d'objet une trajectoire (29) d'interaction prévue pour l'interaction avec l'objet (9) de collision possible en fonction du domaine d'interaction et/ou du point d'interaction et/ou de la direction de rapprochement.

10. Procédé suivant l'une des revendications 7 à 9, **caractérisé en ce que** l'on détermine au moins une partie du paramètre d'objet, en exploitant les données de détection en ce qui concerne l'objet (9) de collision possible.

11. Procédé suivant l'une des revendications précédentes, **caractérisé en ce que** l'on recherche au moins une partie du paramètre de fonctionnement à utiliser en ce qui concerne une classe d'objet sélectionnée et/ou au moins une partie du paramètre d'objet se rapportant à un objet représentatif de la classe d'objet sélectionnée dans une base de données de paramètre mise en mémoire dans un ou dans le dispositif (14) de mise en mémoire de l'unité (1) mobile de la technique médicale et/ou dans un ou dans le dispositif (16) informatique extérieur, base de données qui correspond notamment à la base de données d'informations de référence.

12. Procédé suivant l'une des revendications précédentes, **caractérisé en ce que** l'on utilise comme paramètre de fonctionnement une distance minimum à observer et/ou une vitesse de rapprochement et/ou une courbe de vitesse de rapprochement et/ou une force d'interaction admissible au maximum.

13. Procédé suivant l'une des revendications précédentes, **caractérisé en ce que** pour un objet (9) de collision possible associé à une classe d'objet comprenant des personnes (21), on active, dans le mode de fonctionnement de sécurité permettant le fonctionnement de manière autonome une dynamique amoindrie par rapport à un mode de fonctionnement normal.

14. Procédé suivant la revendication 13, **caractérisé en ce que** l'on utilise des classes d'objets comprenant plusieurs personnes (21), notamment une classe d'objets pour le personnel et/ou une classe d'objets pour des patients et/ou une classe d'objets pour des visiteurs et/ou une classe d'objets pour des enfants, des modes de fonctionnement de sécurité différents étant utilisés pour des pratiques différentes s'occupant de personnes (21) ayant des unités mobiles de la technique médicale.

15. Procédé suivant l'une des revendications précédentes, **caractérisé en ce qu'**au moyen d'un dispositif (12) de détermination de position, on détermine la position en cours de l'unité (1) mobile de la technique médicale dans un environnement de fonctionnement, des informations de propriété, associées à la position en cours, étant recherchées dans une base de données à carte et étant prises en compte dans le fonctionnement de manière autonome de l'unité (1) mobile de la technique médicale.

16. Procédé suivant la revendication 15, **caractérisé en ce que** les informations de propriété comprennent des propriétés statiques pertinentes pour le comportement vis-à-vis d'autres objets et/ou notamment des informations d'état en cours ou mises à jour de manière dynamique dans la base de données à carte.

17. Procédé suivant la revendication 16, **caractérisé en ce que** les propriétés statiques concernent l'adhérence au sol et/ou l'espace de dégagement à disposition et/ou **en ce que** l'on utilise comme information d'état un état d'ouverture d'une porte et/ou des informations décrivant un chantier et/ou un emplacement à problème en cours.

18. Procédé suivant l'une des revendications précédentes, **caractérisé en ce que** l'on utilise comme classe d'objets au moins une classe de personnes et/ou au moins une classe de dispositifs de diagnostic et/ou au moins une classe de dispositifs de transport de patient et/ou au moins une classe de dispositifs de thérapie.

19. Unité (1) mobile de la technique médicale, comportant au moins un dispositif (6) de détection pour la détection d'objets (9) de collision possibles aux alentours de l'unité (1) et un dispositif (2) de commande, qui exploite les données de détection de l'unité (6) de détection et qui est constitué pour le fonctionnement au moins en partie de manière autonome possible de déplacement de l'unité (1), en réalisant une fonction de protection vis-à-vis d'une collision, **caractérisée en ce que** le dispositif (2) de commande est constitué pour effectuer un procédé suivant l'une des revendications précédentes.
